# EUROPEAN PATENT APPLICATION

(11) **EP 0 960 941 A1**
(43) Date of publication of application: **01.12.1999**
(21) Application number: 99113411.5
(22) Date of filing: 10.04.1991
(51) Int. Cl.: C12N 15/86, C07K 14/47, A61K 48/00

(54) **Gene Therapy for cell proliferative diseases**

(30) Priority: 10.04.1990 US 507417
(62) Divisional of application: 91908358.4
(71) Applicant: CANJI, Inc., San Diego, CA 92121 (US)
(72) Inventor: Fung, Yuen Kai, Los Angeles, CA 90039 (US); Murphree, Alan Lim, Los Angeles, CA 90039 (US)
(74) Representative: Wilkinson, Stephen John

(57) **Abstract**

A pharmaceutical composition for the treatment of pathological cellular proliferative diseases by administration to a cell comprises a negative growth regulatory gene which directs the expression of an encoded peptide. Typically the composition comprises DNA which encodes the negative growth regulatory gene which DNA is contained within a recombinant expression vector.

## Description

### Field of the Invention

The present invention relates to a therapeutic modality for treatment of cell proliferative diseases comprising the insertion of a negatively-acting growth regulatory (NGR) element into a proliferating cell.

### Background of the Invention

Pathological conditions resulting in inappropriate local proliferation of cells are a common cause of human disease. Benign human diseases differ from malignant processes (cancer) primarily by the inability to spread from one part of the body to another and their generally slower growth rate. Both can kill, blind and maim. Unwanted internal adhesions and scarring after abdominal surgery can lead to bowel strangulation and death. Blindness from diabetes mellitus results from the inappropriate growth of new blood vessels inside the eye. Benign neurofibromas cause disfiguration. A skin disease like psoriasis is a life-long debilitating process resulting from the inappropriate overgrowth of otherwise normal cells. Scores of different human diseases can be assigned to this category.

Diseases resulting from inappropriate local proliferation of cells will be referred to in the collective as Benign Proliferative Diseases (BPD). When the cell predominating in the overgrowth population is the fibroblast, the diseases in the subgroup are known as "fibroproliferative" disorders. When the predominating cell is vascular endothelium (a cell type which lines the inner surface of blood vessels), the subgroup is known as "vasoproliferative" disorders. The inappropriate overgrowth of epithelium results in serious skin diseases. Currently, treatment of these benign proliferative diseases frequently requires tissue ablation with one or more of the following treatments: surgery, radiation, and/or chemotherapy. These treatments are all generally attended by damage to normal as well as pathological tissues. Other local treatment modalities, such as laser or local cryotherapy, are rarely successful.

### Regulation of Cell Proliferation

The control of cell proliferation is a complex process which has only just begun to be understood in recent years. Whether a cell is to grow or not depends on the balance of the expression of negatively-acting and positively-acting growth elements. Negatively-acting growth regulatory (NGR) elements are those that, when expressed in or provided to a cell, lead to suppression of cell growth. Positively-acting growth regulatory (PGR) elements are generally those products, or genes and their products which, when expressed in or provided to a cell, stimulate its proliferation.

### Negative Growth Regulatory (NGR) Elements

The expression of negatively-acting growth genes is important in regulating and controlling cell proliferation. Examples of NGR elements are several genes which have been identified, the expression of which is induced in cells that are quiescent or in the resting state. These genes may be termed negative growth regulatory (NGR) genes. Down regulation of these negatively-acting growth regulatory genes results in the removal of the inhibition of cell proliferation. In addition to biological or physiological phenomena which result in the inactivation of these genes or the gene products, certain pathological conditions have been identified in which the negatively-acting growth regulatory genes are inactive, or absent. These genes may be inactive due to deletions, mutations or downregulation of the gene, or due to the overabundance of a factor which binds or inactivates the gene product and, therefore, prevents, partially or completely, the expression of the gene or the function of its product, and thus, its ability to block or inhibit cell proliferation.

Recently, several NGR genes called tumor suppressor genes which have a negative effect on tumor cell proliferation have been identified. These genes include, but are not limited to, the human retinoblastoma gene Rb-1, the p53 gene, the DCC gene, the NF1 gene, the Wilms tumor gene, the NM 23 gene, the thyroid hormone receptor gene and the retinoic acid receptor. The absence or inactivation of some of these negative growth regulatory genes has been correlated with certain types of cancer.

The human retinoblastoma gene, Rb1, is the prototype of this class of tumor suppressor genes, herein termed Negative Growth Regulatory (NGR) genes in which the absence of both alleles of the gene in a cell or the inhibition of the expression of the gene or its gene product will lead to neoplastic or abnormal cellular proliferation. It has been demonstrated at the molecular level that the loss or inactivation of both alleles of the RB1 gene is involved in the clinical manifestation of tumors such as retinoblastoma and clinically related tumors, such as osteosarcomas, fibrosarcoma, soft tissue sarcoma and melanoma.

Additionally, loss of the function of the Rb-1 gene has also been associated with other types of primary cancer such as primary small cell lung carcinoma (SCLC), soft tissue sarcomas, breast carcinoma, cervical carcinoma and prostate carcinoma.

Introduction of the Rb-1 gene into a tumor cell that has lost the Rb-1 gene, resulted in the suppression of the growth of the tumor. For instance, when the cDNA of Rb-1 was delivered into the osteosarcoma cell line SAOS2 and the prostate cancer cell line DU 145, tumor cells that have lost their endogenous Rb gene, the growth of some of these tumor cells was specifically suppressed in vivo (Huang et al., (1988) Science 242:1563; Bookstein et al., (1990) Science 247:712). These authors concluded that, while the retinoblastoma gene specifically suppressed the tumorigenic phenotype, it had no effect on normal cells.

### Positively-Acting Growth Regulatory (PGR) Elements

Positively-acting growth regulatory (PGR) elements include all the proto-oncogenes, the expression of which has been shown to be activated in some forms of cancer and during cell proliferation in normal cells and in certain pathological cell proliferative diseases. Activation of the expression of proto-oncogenes is indispensable for cell proliferation. Many of the specific genes in this class have been found to code for growth factors or their receptors. The expression of some proto-oncogenes has been found to be tightly linked to the proliferative state when a cell is exposed to growth factors. Many growth factors, such as platelet derived growth factor (PDGF), epidermal growth factor (EGF), insulin, and transferrin, to name a few, are required for cell proliferation. Cells which do not express the specific receptor molecule for a growth factor cannot proliferate even when the growth factor is presented to the cell. On the other hand, cells in which the normal negative regulation of the expression of the receptor is interfered with, resulting in uncontrolled expression of the receptor, will proliferate in an uncontrolled manner. When quiescent cells are exposed to platelet derived growth factor (PDGF) or epidermal growth factor (EGF), the synthesis of new mRNA of several proto-oncogenes including c-fos and c-myc is induced. Similarly, resting hepatic cells can be stimulated to grow in vivo by partial hepatomy. This stimulation of growth is associated with an elevated expression of several proto-oncogenes including c-myc. Activation of these PGR genes is required for normal, as well as abnormal or pathological, cell proliferation.

Pathological cell proliferation disorders or diseases are often associated with inappropriate activation of proto-oncogenes. The level of c-myc expression, for example, is highly amplified in the non-lymphocytic leukemia cell line HL-60. When HL-60 cells are induced to stop proliferation by chemical inducers, such as retinoic acids and dimethyl sulfoxide, the level of c-myc is downregulated. On the other hand, exposure of HL-60 cells to a DNA construct which is complementary to the 5' end of c-myc or c-myb causes arrest of translation of these mRNAs and c-myc or c-myb protein expression is down regulated, resulting in the arrest of cell proliferation and differentiation of the treated cells (Wickstorm et al (1988) Proc. Natl. Acad. Sci 85: 1028; Anfossi et al (1989) Proc. Natl. Acad. Sci 86: 3379).

### Summary of the Invention

In one aspect, the present invention provides a generalized approach to the treatment of inappropriate or pathological cell growth such as exists in cancer, malignant cell proliferation, benign proliferative diseases and other pathological cell proliferative diseases.

In another aspect, the present invention provides a method of treating cell-proliferative diseases in individuals comprising administration of one or more active gene copies of a negatively-acting growth regulatory gene (NGR) element to an abnormally proliferating cell.

In another embodiment, the present invention provides a method to inhibit abnormal cell proliferation by inserting into an abnormally proliferating cell, a synthetic DNA or RNA construct of the present invention, which construct inactivates the functional expression of a PGR element.

One embodiment of the present invention provides a method of treating cancer and cell-proliferative diseases in individuals comprising administration of one or more active gene copies of a negatively-acting growth regulatory (NGR) gene.

In one aspect, the present invention provides a method for the inhibition of pathological or abnormal cell growth or proliferation by the insertion of one or more copies of a negatively acting growth gene selected from the group consisting of the human retinoblastoma gene, the p53 gene, the p85 gene, the p107 gene, the p130 gene, the retinoic acid receptor gene, the thyroid hormone receptor gene, the myo D gene, the DCC gene, the NF-1 gene, and the NM-23 gene.

In another embodiment, the present invention provides a method for the treatment of pathological cell proliferative diseases comprising administering a ribozyme construct of the present invention to a cell containing a PGR element such as an oncogene or proto-oncogene wherein the encoded ribozyme destroys the pre-mRNA and/or the mRNA of the target PGR gene or element.

In another embodiment, the present invention provides a method for the treatment of pathological cell proliferative diseases comprising administration of one or more NGR elements to an abnormally proliferating cell, wherein said NGR element is selected from the group consisting of (1) a negatively-acting growth regulatory gene, and (2) DNA encoding a ribozyme specifically directed against the pre-mRNA or mRNA of a PGR gene or element.

In another embodiment, the present invention provides a method for the treatment of pathological cell proliferative diseases comprising administering an NGR element to a cell containing a PGR gene, wherein said NGR element interferes with or inhibits the normal functional expression of the PRG element.

It was a further object of the present invention to provide a method for the treatment of pathological cell proliferative diseases comprising administering a DNA construct comprising an NGR element to a cell containing an oncogene or proto-oncogene wherein said DNA construct represses the activation of the oncogene, proto-oncogene or other PGR element or inactivates the gene product of said oncogene, proto-oncogene or other PGR element wherein said DNA construct comprises a recombinant expression vector effective in expressing a DNA sequence encoding a ribozyme operably linked to a second control DNA sequence compatible with a transformant host cell. In a preferred embodiment, the DNA construct comprises a DNA sequence encoding said ribozyme wherein said ribozyme contains sequences complementary to the nuclear or messenger RNA or the gene product of an oncogene, proto-oncogene or other PGR element present in the transformant host cell. The method of the present invention may be utilized to inactivate the pre-mRNA or mRNA of any PGR element and, preferably, inactivates a PGR element selected from the group consisting of c-myc, c-fos, c-jun, c-myb, c-ras, Kc and JE, transferrin receptor, insulin receptor, PDGF receptor, and the EGF receptor. In a most preferred embodiment the DNA construct of the present invention is operably linked to an inducible promotor.

In another embodiment, the present invention provides a method of treating cell-proliferative diseases in individuals comprising administration of one or more active gene copies of an NGR gene to a cell wherein said NGR gene is selected from the group consisting of the retinoblastoma gene, the p53 gene, the p85 gene, the p107 gene, the p130 gene, the retinoic acid receptor, the thyroid hormone receptor, the myo D gene, the DCC gene, the NF-1 gene, and the NM-23 gene. In a preferred embodiment the negative growth regulatory gene is a DNA construct comprising a recombinant expression vector effective in expressing a DNA sequence encoding said negative growth regulatory gene. Preferably said DNA construct is operably linked to a second control DNA sequence compatible with a transformant host cell.

In another aspect, the present invention provides a method for inhibiting abnormal cell proliferation by inserting an NGR element selected from the group consisting of an anti-oncogene, a negative growth regulatory gene, and DNA construct encoding a ribozyme directed against a positively-acting growth regulatory gene or element into abnormally proliferating cells.

In one embodiment, the NGR element is the retinoblastoma gene, Rb-1.

Another aspect of the present invention provides a method for the prophylactic treatment to inhibit and prevent abnormal cell proliferative diseases, such as cancer, in genetically predisposed individuals comprising the steps of identifying individuals having only one active allele of an NGR gene and, thus, predisposed to tumor development, inserting at least one additional copy of a negative growth gene selected from the group consisting of the human retinoblastoma gene, the p53 gene, the p85 gene, the p107 gene, the p130 gene, the retinoic acid receptor gene, the thyroid hormone receptor gene, the myo D gene, the DCC gene, the NF-1 gene, and the NM-23 gene, into the egg, sperm, or fertilized egg of said predisposed individual identified as lacking sufficient normal copies of said negative growth genes and allowing fertilization or continued embryological development to continue.

In a preferred embodiment of the present invention, the NGR element is inserted into proliferating cells utilizing a retrovirus vector. In a most preferred embodiment, the retroviral vector is defective and will not transform non-proliferating cells.

It was a further aspect of the present invention to provide a method for the treatment of pathological cell proliferative diseases such as, but not limited to, proliferative intraocular disorders, proliferative skin diseases, such as but not limited to, psoriasis, ichthyosis, lichen plannus, papillomas, basal cell carcinomas, squamous cell carcinoma, vasoproliferative diseases of all organs, and other similar proliferative cell diseases throughout the body.

Other and further aspects, features, and advantages of the present invention will be apparent from the following description of the presently preferred embodiments of the invention given for the purpose of disclosure.

### Brief Description of the Figures

Figure 1 shows the construction and the structure of plasmid HuBAcpr-1-neo-Rb containing part or all of the Rb-1 cDNA under the control of a human β actin promoter.
Figure 2 demonstrates the effect of transfected plasmids on proliferation of the human fibroblast cell line WS1.
Figure 3 demonstrates the effect of transfected plasmids on proliferation of the human bladder carcinoma cell line TCCSUP.
Figure 4 demonstrates the effect of transfected plasmids on proliferation of osteosarcoma SaOS2 cells in vitro and in vivo.
Figure 5 shows a schematic representation of a ribozyme DNA construct. Panel 5A shows a general schematic for a ribozyme DNA construct, Panel 5B shows the DNA construct encoding a ribozyme targeted to Rb RNA, Panel 5C shows Rb-1 containing plasmid PT7B-Rb-FL used to generate the two RNA templates for testing the catalytic activity of the Rb-ribozyme and Panel 5D shows the DNA construct encoding a ribozyme targeted to c-myc mRNA.
Figure 6 shows the schematic representation of the retroviral vector containing one or more ribozyme (Panel 6A), antisense (Panel 6B), and NGR gene (Rb-1 gene) (Panel 6C).
Figure 7 demonstrates the electrophoretic pattern of two Rb synthetic RNA templates before and after treatment with a targeted ribozyme.
Figure 8 demonstrates the electrophoretic pattern of various proteins precipitated by Rb antibodies.

### Detailed Description of the Preferred Embodiments Definitions

The term "negatively-acting growth regulatory element" (NGR element) is meant to include agents which decrease cell growth, or inhibit the proliferation of cells when administered to proliferating cells and is incorporated into the genome of said cells. One specific embodiment of this invention is a DNA construct of an NGR gene, the Rb-1 gene, incorporated into a vehicle or vector preferably under the promotion of a strong promoter. Preferably the DNA construct of the present invention is delivered to the target cells utilizing the MuLV based retroviral vector.

The term NGR element is meant to include any gene, gene coding region or gene product, any natural or synthetic DNA or RNA, peptide, steroid, or other biological material which, when present in or provided to a cell, contributes to the suppression of cell growth or proliferation.

For instance one type of NGR element is recessive human cancer genes (tumor suppressor genes or anti-oncogenes) selected from the group consisting of but not limited to the retinoblastoma gene Rb-1, the p53 gene, the DCC gene, the NF-1 gene, the Wilms' tumor gene, the NM 23 gene, the thyroid hormone receptor gene, the retinoic acid receptor gene, and the genes that encode p130, p107 and p85.

A second type of NGR element is a DNA construct encoding a ribozyme. Said DNA construct is designed to produce an RNA enzyme which binds and destroys the RNA of selected positively-acting growth regulatory (PGR) genes such as (1) oncogenes or proto-oncogenes selected from the group consisting of, but not limited to, the following: c-myc, c-fos, c-jun, c-myb, c-ras, Kc and JE; (2) growth factor receptor genes selected from the group consisting of but not limited to platelet derived growth factor (PDGF), epidermal growth factor (EGE), transferrin and insulin.

The term "negatively-acting growth regulatory (NGR) gene" is meant to include that subgroup of NGR elements which consists of a gene or gene coding region selected from the group consisting of, but not limited to, the retinoblastoma gene RB-1, the p53 gene, the p85 gene, the p107 gene, the p130 gene, the DCC gene, the NF-1 gene, the Wilms' tumor gene, the NM 23 gene, the thyroid hormone receptor gene and the retinoic acid receptor gene.

The term "positively-acting growth regulatory element" is meant to include any gene or gene product, any natural or synthetic DNA or RNA, peptide, steroid, or other biological material which, when present in or provided to a cell, contributed to the stimulation of cell growth or proliferation. PGR elements may be selected from the group consisting of, but not limited to, oncogenes, such as viral oncogene vsrc, proto-oncogenes such as c-myc, growth factor genes c-sis, growth factor receptor genes such as PDGF receptor gene, growth factors such as insulin, growth factor receptors such as the insulin receptor or the transferrin receptor.

The term "functional expression of the gene" is meant to include the suppression of transcription of the gene, the degradation of the gene transcript (pre-message RNA), the inhibition of splicing, the destruction of the message RNA, the prevention of translation of the message RNA, the prevention of the post-translational modifications of the protein, the destruction of the protein, or the inhibition of the normal function of the protein.

The term "ribozyme" is meant to include an RNA constructed to recognize and bind with the mRNA or pre-mRNA of the target gene of choice, which ribozyme then cleaves or otherwise destroys said pre-mRNA or mRNA. Ribozymes can be constructed to be targeted against the pre-mRNA or mRNA of any gene if the DNA sequence of that gene is known.

The term "transfected plasmid" is meant to include the bacterial plasmid which contains the NGR element of choice selected from, but not limited to, the group consisting of (1) NGR gene or part of the gene, (2) ribozyme constructed to destroy the PremRNA and/or mRNA of a PGR element, to be carried (transfected) into the cell of choice.

The term "gene therapy" is meant to include the insertion of part or all of a gene, a DNA construct, RNA, or gene product into a cell, group of cells, tissue, pathologic lesion, organ or organism for the purpose of modulating gene expression, and/or function of the gene product.

The term "prophylactic gene therapy" is meant to include genes which may be used for partial or total inhibition or prevention of disease and the spread of disease and also is meant to include genes which may be used to supplement or replace absent or defective negative growth in cell, tissues or germlines.

The term "cell proliferative disease" is meant to include any human or animal disease or disorder, affecting any one or any combination of organs, cavities or body parts, which is characterized by single or multiple local abnormal proliferations of cells, groups of cells or tissue(s), whether benign or malignant.

The term "prokaryote" is meant to include all bacteria which can be transformed with the DNA for the expression of the recombinant molecules of the present invention.

The term "eukaryote" is meant to include all yeasts, fungi, animal and plant cells which can be transformed with the DNA for the expression of the recombinant molecules of the present invention.

The DNA for DNA constructs of the present invention can be synthetic or may be derived from any mammalian species. All that is required is that the genetic sequence for the NGR elements be functionally expressed in the prokaryotic or eukaryotic organism. Preferred is synthetic DNA.

A recombinant DNA molecule coding for the DNA constructs of the present invention can be used to transform a host using any of the techniques commonly known to those of ordinary skill in the art.

Methods for preparing fused, operably linked genes and expressing them in bacteria are known and are shown, for example, in U.S. Patent No. 4,366,246, herein incorporated by reference. The genetic constructs and methods described therein can be utilized for construction of the DNA constructs of the present invention and transfection in prokaryotic or eukaryotic hosts.

Prokaryotic hosts may include Gram negative as well as Gram positive bacteria, such as E.coli, S. tymphimurium, Serratia marcescens, and Bacillus subtilis.

Eukaryotic hosts may include yeasts such as Pishia pastoris or mammalian cells.

In general, expression vectors containing promoter sequences which facilitate the efficient transcription of the inserted DNA fragment are used in connection with the host. The expression vector typically contains an origin of replication, promoter(s), terminator(s), as well as specific genes which are capable of providing phenotypic selection in transformed cells. The transformed hosts can be fermented and cultured according to means known in the art to achieve optimal cell growth.

Examples of promoters which can be used in the invention include, but are not limited to: human β actin promotor, metallothionin promotor, SV40 origin of replication, and MMTV LTR promotor and MuLV LTR promotor. Examples of some of the plasmids or bacteriophage which can be used in the invention are listed in Maniatis et al., Molecular Cloning, Cold spring Harbor Laboratories, 1982, and others are known to those of skill in the art and can be easily ascertained.

A gene is a DNA sequence which encodes through its template or messenger RNA a sequence of amino acids characteristic of a specific peptide. The term cDNA includes genes from which the intervening sequences have been removed. The term "recombinant DNA" (rDNA) is meant to include a molecule that has been recombined by splicing cDNA or genomic DNA sequences in vitro.

A cloning vehicle is a plasmid or phage DNA or other DNA sequence which is able to replicate in a host cell which is characterized by one or a small number of endonuclease recognition sites at which such DNA sequences may be cut in a determinable fashion without loss of an essential biological function of the DNA, and which contains a marker suitable for use in the identification of transformed cells. Markers, for example, are tetracycline resistance or ampicillin resistance. The word "vector" is sometimes used for a cloning vehicle.

An expression vehicle is a vehicle similar to a cloning vehicle but which is capable of expressing a given structural gene in a host, normally under control of certain control sequences.

The term "individual" is meant to include animals and humans.

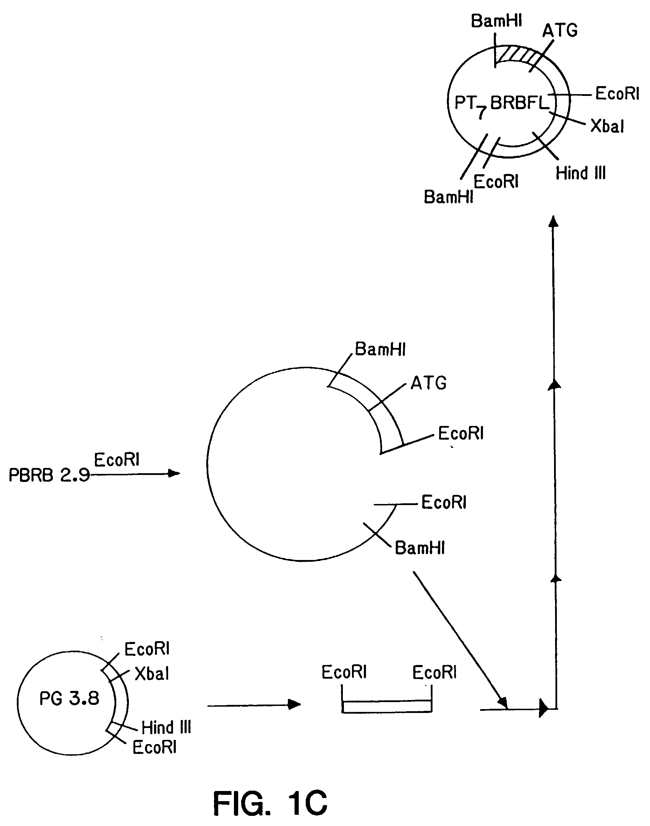

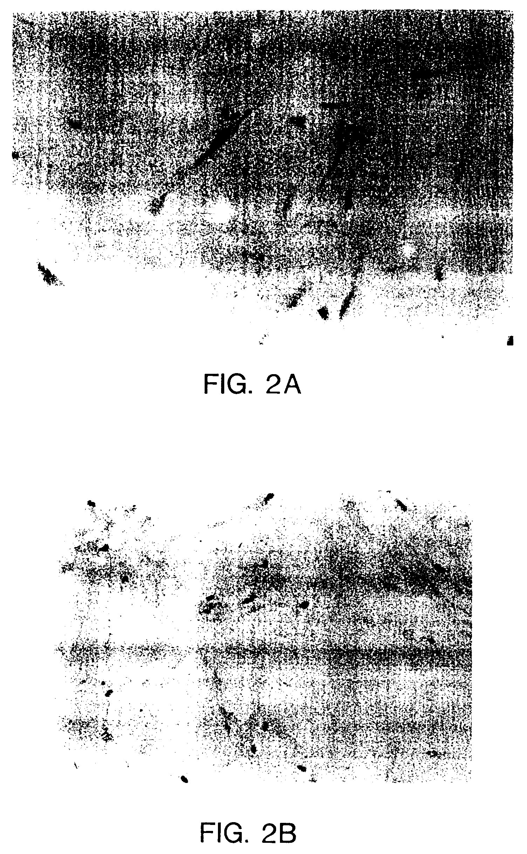

The term "biologically inhibiting" or "inhibition" of the growth of proliferating cells is meant to include partial or total growth inhibition and also is meant to include decreases in the rate of proliferation or growth of the cells. The biologically inhibitory dose of the NGR elements or genes of the present invention may be determined by assessing the effects of the test NGR element on target malignant or abnormally proliferating cell growth in tissue culture (see Examples 3-4 and Figures 2-4), tumor growth in animals and cell culture or any other method known to those of ordinary skill in the art.

Administration of the NGR elements or genes useful in the method of the present invention may be by topical, intraocular, parenteral, oral, intranasal, intravenous, intramuscular, subcutaneous, or any other suitable means. The preferred method of administration for treatment of ocular diseases is intraocular or periocular injection. The preferred method of administration for treatment of skin cell proliferative diseases is by topical application or subcutaneous injection.

In another embodiment, the DNA constructs of the present invention may be delivered to the focal proliferative disease directly. In the case of ocular proliferative disease, the DNA constructs of the present invention may be directly injected into the eye. The DNA constructs of the present invention may be delivered directly to focal disease sites in internal organs, body cavities and the like by use of imaging devices used to guide the injecting needle directly to the disease site. The DNA constructs of the present invention may also be administered to disease sites at the time of surgical intervention.

The DNA dosage administered is dependent upon the age, clinical stage and extent of the disease or genetic predisposition of the individual, location, weight, kind of concurrent treatment, if any, and nature of the pathological or malignant condition. The effective delivery system useful in the method of the present invention may be employed in such forms as capsules, tablets, liquid solutions, suspensions, or elixirs, for oral administration, or sterile liquid forms such as solutions, suspensions or emulsions. Any inert carrier is preferably used, such as saline, or phosphate-buffered saline, or any such carrier in which the compounds used in the method of the present invention have suitable solubility properties.

Preferably, for intraocular delivery and for delivery to other localized disease sites, delivery systems useful in the method of the present invention may be employed in such sterile liquid forms such as solutions, suspensions or emulsions. For topical use it may be employed in such forms as ointments, creams or sprays. Any inert carrier is preferably used, such as saline, or phosphate-buffered saline, or any such carrier in which the compounds used in the method of the present invention have suitable solubility properties.

For local administration of the NRG elements or genes to cells, the DNA of the present invention may be administrated by any method known to those of skill in the art including, but not limited to, transfection, electroporation, microinjection of cells, or in vehicles such as liposomes, lipofectin or as naked DNA or RNA. The DNA of the present invention may be delivered by known gene delivery systems such as, but not limited to, retroviral vectors (Gilboa (1982) J. Virology 44:845; Hocke (1986) Nature 320:275; Wilson et al., Proc Natl Acad Sci USA 85:3014, vaccinia virus system (Chakrabarty et al., (1985) Mol. Cell Biol. 5:3403) or other efficient DNA delivery systems (Yates et al., (1985) Nature 313:812) known to those of skill in the art. These references are exemplary only and are incorporated herein by reference. In order to specifically deliver or transfect cells which are abnormally proliferating and spare non-dividing cells, it is preferable to utilize a retrovirus delivery system known to those of skill in the art. Since host DNA replication is required for retroviral DNA to integrate and the retrovirus will be unable to self replicate due to lack of the retrovirus genes needed for its life cycle, utilizing such a retroviral delivery system for the NGR elements of the present invention will target said anti-proliferative NGR elements to abnormally proliferating cells and will spare non-dividing normal cells from any intervention.

The DNA of the present invention may be administered in any biologically effective carrier. The biologically effective carriers may include retroviruses, liposomes, and any other transfection mechanism capable of introducing foreign DNA into the genome of the cell. Such transfection mechanisms are known to those of skill in the art. The carrier may also include any agent or solvent with which the constructs of the present invention are compatible and which are non-toxic to the individuals or cells treated at the amounts administered.

There is a wide variety of pathological cell proliferative conditions for which the method of the present invention will provide therapeutic benefits. These pathological conditions may occur in almost all cell types capable of abnormal cell proliferation. Among the cell types which exhibit pathological or abnormal growth are (1) fibroblasts, in which case the diseases are known as fibroproliferative disorders; (2) vascular endothelial cells, in which case the diseases are known as vasoproliferative disorders and (3) epithelial cells in which case the diseases are known as dermatoproliferative diseases. It can be seen from the above that the method of the present invention is useful in treating local or disseminated pathological conditions in all or almost all organ and tissue systems of the individual.

For instance, in the eye alone, the method of the present invention may be utilized to treat such a wide variety of pathologic disease states which are due to abnormal proliferation of normal, benign or malignant cells or tissues including, but not limited to, the following fibroproliferative, vasoproliferative and/or neoplastic diseases of the eye: retinopathy of prematurity, proliferative vitreoretinopathy, proliferative diabetic retinopathy, capillary hemangioma, choroidal neovascular nets, subretinal neovascular nets, senile macular degeneration due to subretinal, neovascularization, corneal neovascularization, macular pucker due to epiretinal membrane proliferation, adult cataracts due to nuclear sclerosis, fibrous ingrowth following trauma or surgery, optic nerve gliomas, angiomatosis retinae, neovascular glaucoma, cavernous hemangioma, rubeosis iridis, sickle cell proliferative retinopathy, epithelial downgrowth after eye surgery or injury, after-cataract membrane, papilloma, retinal neovascularization in thalassemia, subretinal neovascularization due to pseudoxanthoma elasticum, and neurofibromatosis type l and 11, retinoblastoma, uveal melanoma, and pseudotumor of the orbit.

Other benign cell proliferative diseases for which the present invention is useful include, but are not limited to, psoriasis, ichthyosis, papillomas, basal cell carcinomas, squamous cell carcinoma, and Stevens-Johnson Syndrome.

According to the method of the present invention, one can manipulate the cellular proliferative process by the introduction of an NGR element or multiple NRG elements to prevent or inhibit abnormal proliferation in a wide variety of cell proliferative diseases. The manipulation of the proliferative process may be accomplished by introducing into a target proliferating cell a DNA construct which encodes an NGR element. This NGR element comprises either an NGR gene or inactivates or inhibits the functional expression of a PGR element.

To illustrate the use of an NGR element to inhibit abnormal cell proliferation in non-malignant or non-cancer cells by the method of the present invention, the human retinoblastoma gene, Rb-1, was introduced into a normal human fibroblast cell line. As described more fully in Example 2, introduction of a human Rb cDNA into normal human fibroblast, WS1, led to the cessation of cell growth. Similarly, introduction of this human retinoblastoma gene into several different tumor cell types led to the suppression of growth of these cells in vitro or in vivo as further described in Examples 3 and 4.

We and others have shown that the human retinoblastoma gene product is heavily phosphorylated when cells are proliferating at the G1/S boundary and the S phase of the cell cycle (Buckovich et al., (1989) Cell 58:1097; Mihara et al., (1989) Science 246:1300; Decaprio (1989) Cell 58:1085 and Chen et al., (1989) Cell 58: 1193), but is unphosphorylated or underphosphorylated when cells are not proliferating at the G1 phase. Exposure of a resting cell to mitogens results in the activation of a kinase complex which phosphorylates the Rb protein. As a result, the cell enters into DNA replication and mitosis. Thus, in order for a cell to initiate DNA replication, inactivation of the human retinoblastoma gene product by phosphorylation may be necessary.

In one embodiment of the present invention, the normal cellular phosphorylation of the Rb-1 protein can be inhibited or interfered with by either introduction of additional copies of the RB-1 gene, causing over production of the Rb-1 protein, or by mutagenizing the Rb-1 gene so that it cannot be phosphorylated.

When a highly expressed retinoblastoma gene was introduced into a normal non-tumor cell, abundant amounts of the retinoblastoma protein were expressed. The treated cell therefore contains an excessive amount of active Rb protein and proliferation is arrested. The retinoblastoma protein which normally exists in a multiply phosphorylated form becomes dephosphorylated when proliferating cells were growth arrested by serum depletion. This excessive amount of Rb protein may overwhelm the capacity of the cellular kinase to phosphorylate and inactivate the Rb gene product. Thus, in one embodiment, cell proliferation may be arrested or inhibited by the introduction of NGR genes, such as the Rb-1 gene, into a cell. Similarly, other NGR genes may also be used to practice the present invention. In order to insure the growth arrest process, the genes to be introduced should be placed under the control of a strong promoter. The human β-actin promoter is one such strong promotor. The construction of a DNA construct comprising the Rb gene under the control of a strong promotor such as the human β actin gene is shown in Example 1. However, it will be obvious to one of skill in the art that other NGR elements may be substituted for the RB-1 gene and other strong promotors may also be utilized in practicing the present invention.

In another embodiment, the phosphorylation site the Rb-1 gene is mutagenized before introduction into a cell. There are 7-12 potential phosphorylation sites on the Rb protein. Mutation of the serine or threonine coding sequences in the Rb-1 cDNA into alanine or valine or others would therefore lead to the production of a permanently active Rb protein which cannot be inactivated by phosphorylation. Thus, the host cell will not be able to inactivate the Rb protein by phosphorylation. Introduction of such a mutated Rb-1 gene into a cell will therefore lead to growth arrest. Alternatively, the phosphorylation sites of other NGR genes, such as, but not limited to, P53, P85, P107, and P130 may be mutagenized to prevent phosphorylation of the gene product and introduced into abnormally proliferating cells to inhibit the proliferation of these cells.

An alternative method to arresting cell growth by the introduction of extra copies of an NGR gene is to disrupt the expression of positively-acting regulatory elements or gene products. Natural RNA molecules which act as enzymes in the metabolism of RNA have been identified. This class of molecules, collectively termed ribozymes, has been found in plant and animal viruses. One such ribozyme has been identified in satellite tobacco ringspot virus (STobRV) and a so-called hammer head structure for the self-cleaving RNA has been proposed by Symons and coworkers (Forster and Symons (1987) Cell 50:9). Uhlenbeck has shown that an RNA fragment consisting of a short stretch of RNA mimicking the hammer-head structure can act as an enzyme in the cleavage of a synthetic RNA fragment under physiological conditions. A ribozyme specific for the chloramphenicol acetyltransferase (CAT) RNA was shown to be effective in cleavage of the CAT RNA in vitro (Haseloff and Gerlach, (1988) Nature 334:585) and the same construct, when engineered into an expressive vector, cleaved the CAT RNA at the intended site in vivo (Cameron and Jennings (1989) Proc. Natl. Acad. Sci 86:9139). Moreover, the CAT expression was suppressed in the transfected cells. The cleavage specificity of a ribozyme made of the catalytic domain of STobRV is very broad. It can cut at the end of the triplet 5' GUX 3' where X can either be A, C or U. The specificity of an engineered ribozyme lies in the sequences flanking the catalytic domain. These sequences are chosen to be complementary to the sequence surrounding the site on the RNA to be cleaved.

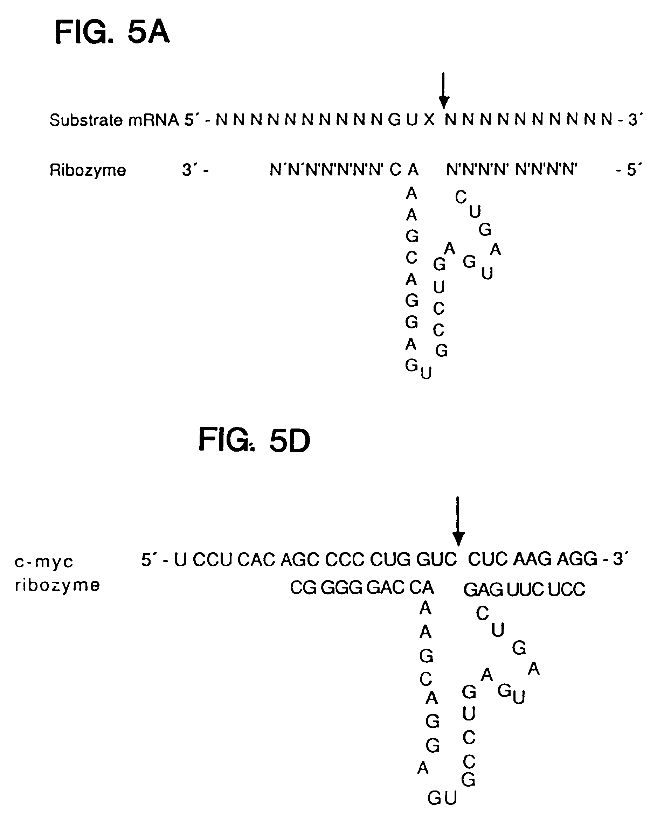

In another embodiment, the present invention provides a method to stop cell proliferation by introducing one or more DNA plasmids which code for the production of ribozymes in a cell specific for the destruction of a PGR element. This embodiment is exemplified in Example 5. The construction of a DNA construct encoding the ribozyme and further comprising sequences to target the ribozyme to any specific PGR element is shown as described in Example 5 and such a ribozyme construct is schematically presented in generalized form in Figure 5A. The construction of a ribozyme which cleaves the RB-1 gene is also described in Example 5. According to one embodiment of the present invention, a ribozyme construct can be introduced into a cell to destroy the nuclear or messenger RNA of the target PGR element. This aspect is exemplified by the c-myc ribozyme described in Example 5. It will be apparent to one of skill in the art that the teachings herein may be applied to any PGR element, and ribozyme constructs can be prepared by the teachings of the present invention to cleave any PGR element or gene product. An alternative means to suppress the expression of a gene in a cell is by means of antisense RNA. Antisense RNAs are RNAs that are a complimentary to part or all of the messenger RNA of the intended gene. Overproduction of antisense RNA has been shown to prevent the use of the target RNA for translation and thereby blocks the expression of the gene product.

In order to stop a cell from proliferating, DNA constructs can be introduced into proliferating cells which express either antisense and/or ribozymes specifically targeted to a variety of positively acting regulatory elements in the cells.

One can administer more than one of the DNA constructs of the present invention simultaneously to the same site. For instance, different ribozyme constructs targeting several different PGR genes can be administered at the same time to the same focal diseased site. Additionally, several different DNA constructs such as one or more ribozymes and one or more NGR genes or NGR elements maybe administered simultaneously.

The present invention also provides a method for the prophylactic treatment to inhibit and prevent cancer in genetically predisposed individuals comprising the steps of: identifying individuals having only one active allele of a negative growth regulator gene and thus being predisposed to tumor development; inserting at least one copy of a negative growth regulatory gene selected from the group consisting of the human retinoblastoma gene, the p53 gene, the p85 gene, the p107 gene, the p130 gene, the retinoic acid receptor, the thyroid hormone receptor, the myo D gene, the DCC gene, the NF-1 gene and the NM-23 gene into the egg, sperm, or fertilized egg of said predisposed individual identified as lacking sufficient normal copy(s) of said negative growth genes; and allowing fertilization or continued embryological development to continue.

Having now generally described the invention, a more complete understanding can be obtained by reference to the following specific examples. These examples are provided for purposes of illustration only and are not intended to be limiting unless otherwise specified.

### Example 1

### Construction of the Rb-1 cDNA Expression Plasmid

The general procedure for plasmid construction and DNA preparation was as described by Maniatis et al. (Maniatis, T., Fuitsch, E.F., and Senbrook, J. (1982) Molecular Cloning: A Laboratory Manual (Cold Spring Harbor Laboratory, Cold Spring Harbor, N.Y., U.S.A.). All plasmids were grown in the Escherichia coli host DH5-α purchased from Bethesda Research Laboratory (BRL). The structure and properties of the human β-actin expression vector system, pHBAPr-1-neo has been described (Gunning et. al., (1987) Proc. Natl. Acad. Sci. USA 84, 4831-4835). The Rb-1 cDNA sequence has been described previously (Fung et al., (1989) in Oncogenes, Chapter 13, Molecular Biology of the Human Retinoblastoma Gene, Benz, and Lin, (eds.) Kluwen Academic Publishers.)

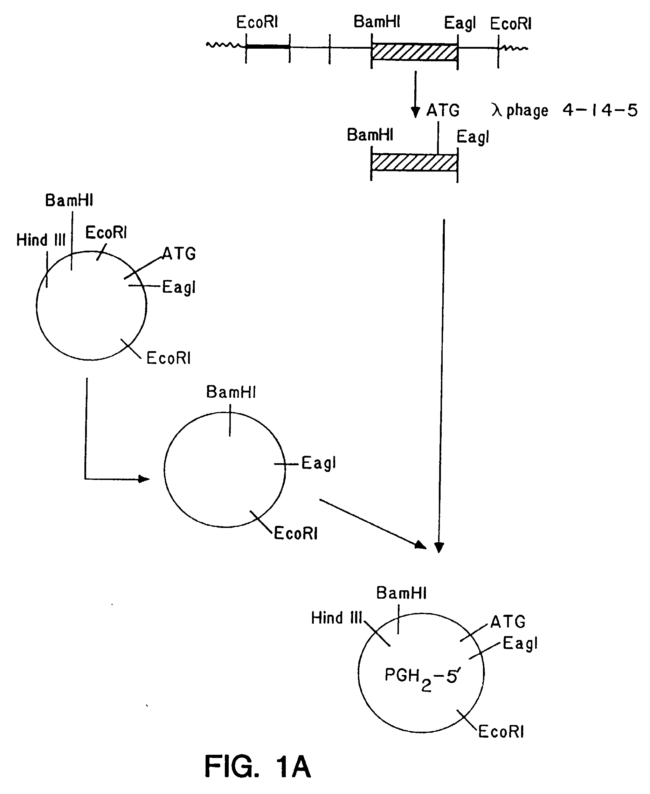

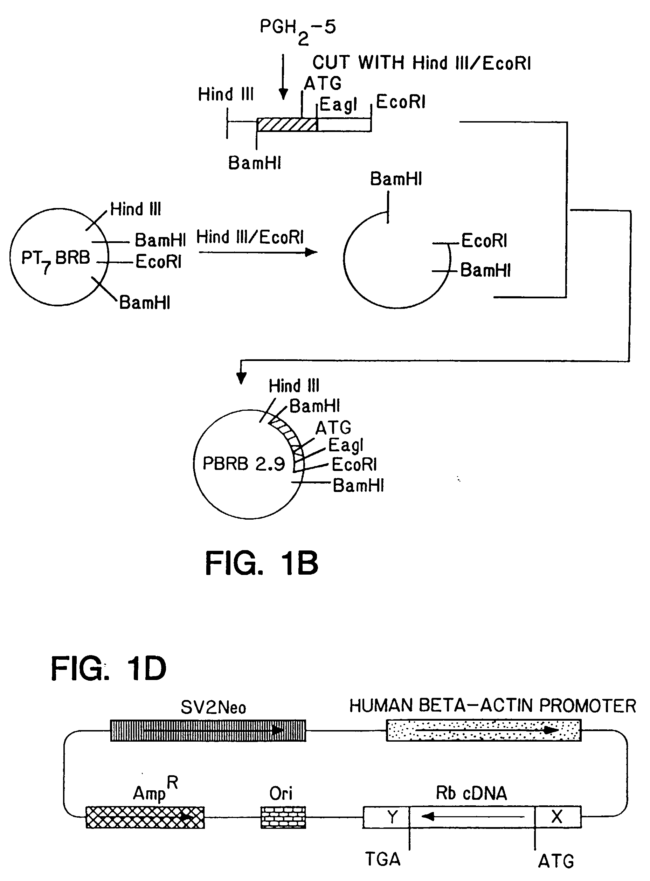

The construction of the HuBAcpr-1-neo-Rb-8342 is schematically demonstrated in Figure 1, Panels A-C. For the construction of HuBAcpr-1-neo-Rb-8342, a BamH1-Eag1 DNA fragment containing part of the first exon was excised from the λ phage clone 4-14-5 (T'Ang et al (1989) Oncogene 4, 401-407) and ligated to the 5' end of Rb-1 cDNA plasmid PGH2 (Fung et al, (1987) Science 236:1657-1661) at the BamH1-Eag1 sites. (See Figure 1, Panel A) The resulting plasmid was grown up and the Hind III EcoR1 fragment containing the Rb-1 promotor region and the 5' portion of the Rb-1 cDNA was transferred to a plasmid PT7BRB at the Hind III and EcoR1 site (See Figure 1, Panel B), generating the plasmid pBRB2.9. The 3.8 kb 3' half of the Rb cDNA was excised with EcoR1 from PG 3.8 (Fung et al (1987) Science 236:1657-1661) and subcloned into the EcoR1 site of pBRB2.9 to generate PT7BRBFL. (See Figure 1, Panel C) Finally, the BamH1 fragment containing the Rb-1 cDNA and its promotor was subcloned into the HBApr-1-neo at the BamH1 site to generate HuBAcpr-1-neo-Rb-8342.

For the construction of HuBAcpr-1-neo-RB-PQ, a double stranded synthetic linker (in which only one strand is shown here):
5' ACGGATC CGCGTC ATG CCG CCC AAA ACC CCC CGA AAA ACG GCCG 3'
was digested with BamH1 and Eag1 and subcloned into the BamH1/Eag1 site of the plasmid PT7BRBFL (See Figure 1, Panel C), replacing the Rb promotor. This plasmid was then partially digested with the enzyme ppum1 to linearize at nucleotide # 2797-2799. A synthetic linker containing a poly A signal (AATAAA) and a BamH1 site flanked by ppum1 sites was inserted into the ppum1 site of the linearized PT7BRBFL. The resulting plasmid was grown up, and the BamH1 fragment containing the Rb1 cDNA coding region was excised and then inserted into the BamH1 site of the plasmid HBAcpr-1-neo to generate the plasmid HuBAcpr-1-neo-Rb-PQ.

Figure 1D shows the general structure of plasmid HuBAcpr-1-neo-Rb containing part or all of the Rb-1 cDNA under the control of a human β-actin promoter.

Two different HuBAcpr-1-neo-Rb expression plasmids were constructed. For HuBAcpr-1-neo-Rb-8342, X is the BamH1 2kb fragment in front of the first ATG of the longest open reading frame (T'Ang et al., (1989) Oncogene 4:401) and Y is the entire 3' untranslated region of Rb cDNA.

For HuBAcpr-1-neo-Rb-PQ, X is a synthetic linker (5-GATCCGCGTC-3) placed in front of the first ATG and Y is a synthetic linker containing a poly A tail signal AATAAA, behind nucleotide #2801.

### Example 2

### Effect of Transfected Plasmids on Proliferation of the Human Fibroblast Cell Line WS1

When normal keratinocytes are exposed to the tumor growth factor β (TGFβ) which inhibits the growth of many cell types, the retinoblastoma protein in the keratinocyte rapidly becomes dephosphorylated and the keratinocyte ceases to grow. The SV 40 large T antigen, which presumably inactivates the retinoblastoma gene product by binding to it, can only bind to the under-phosphorylated (active) form of the retinoblastoma protein. Therefore, as a cell is induced into the growth arrest state, an associated change is the dephosphorylation of the retinoblastoma protein. Restimulation of arrested cells to grow by exposure to mitogens results in heavy phosphorylation of the retinoblastoma protein prior to the initiation of DNA synthesis. The fact that this is observed in normal and tumor cells suggests that the inactivation of the retinoblastoma protein by phosphorylation is required for cell proliferation to occur. The retinoblastoma protein may therefore be involved in the growth retardation or arrest of normal and tumor cells.

To study effect of the RB CDNA on cell growth, HuBAc-1-neo-Rb-PQ was transfected into the normal human fibroblast cell line WS1. As a control, a DNA fragment containing the neomycin resistant gene under the control of the RSV LTR was excised from the plasmid PATV-6D3A, synthetic sal 1 linkers were ligated to the fragment, digested with sal 1 and then ligated to PPVU0 (Kalderon and Smith, (1984) Virology 139:109-137) to generate PPVUO-Neo. This plasmid, PPVUO-Neo, which contains the gene for the SV4O large T antigen was then transfected in order to monitor the efficiency of transfection. For transfection, 100ug of each of the plasmid DNA were mixed with 10⁷ exponentially grown WS1 cells in a final volume of 0.8ml of RPM1 1640 medium (Gibco) plus 10% FCS in an electroporation chamber unit Cell-Porator™ (BRL). Electroporation was done at 200 volt and 1180uF. The electroporated cells were allowed to recover at room temperature for 10 minutes and were then diluted in RPM1 1640 plus 10% fetal calf serum (FCS) and plated out in 60 mm dishes at a cell density of 2 X 10⁴ cells/cm². The cells were allowed to attach and grow in the same medium for two days. Thereafter, the medium was changed to RPM1 1640 + 10% FCS + 15ug/ml G418(GIBCO). Every three days, duplicate dishes were taken for histoimmunochemical staining using either a rabbit polyclonal anti-Rb protein antibodies RB1-ABA1 or RB1-AB18 (Mihara K. et al (1989) Science 246:1300) (Figure 2A), or with the mouse monoclonal anti-SV40 large T antigen antibody PAB 101 (Figure 2B). The staining procedure described in Example 8 was utilized. As can be seen from Figure 2A, 13 days after transfection and selection in G418 medium, the WS1 cells that were expressing the transfected Rb cDNA plasmid HuBAcpr-1-neo-Rb-PQ stained intensely with the anti-Rb protein antibodies. However, the cells that expressed the Rb protein remained as single cells and did not divide. In contrast, cells that expressed the transfected SV40 large T antigen (SVLT) continued to divide into colonies as shown by the group of cells stained positive with the mouse anti SVLT antibody in Figure 2B.

Thus, over expression of the Rb cDNA in a cell led to extreme retardation or arrest of cell growth.

### Example 3

### Effect of Transfected Plasmids on Proliferation of the Human Bladder Carcinoma Cell Line TCCSUP

The Rb-1 gene was transfected into the human bladder carcinoma cells TCCSUP which has no endogenous Rb protein using the same procedures described in Example 2. The plasmids used, the methodology for transfection and immunostaining are identical to those shown in Figure 2.

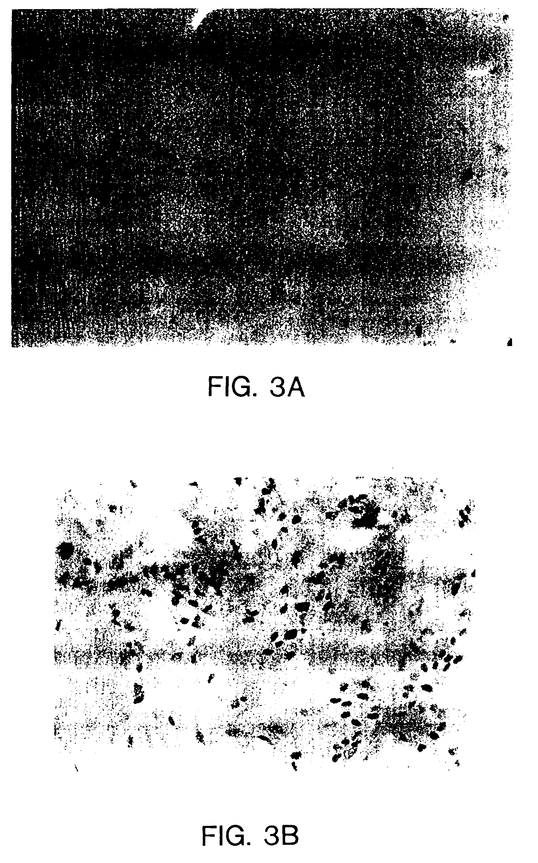

As can be seen in Figure 3A, cells expressing the transfected R-1b cDNA failed to divide. In contrast, cells expressing the transfected SVLT divided to form colonies (Figure 3B). Overexpression of the Rb cDNA led to severe retardation of growth of the tumor cells in vitro.

### Example 4

### Effect of Transfected Plasmids on Proliferation of the Osteosarcoma SaOS₂ Cell Line

The plasmids and the conditions for transfection of the SaOS₂ cells were identical to those described in Example 2 with the exception that 100 ug/ml G418 was used in the medium.

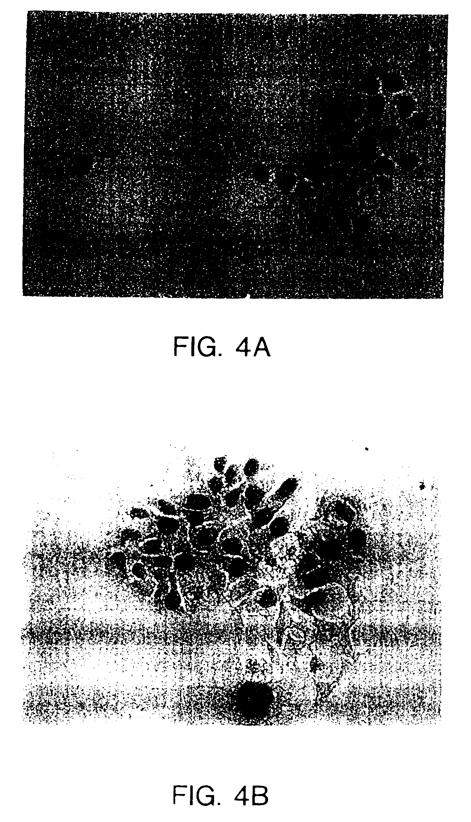

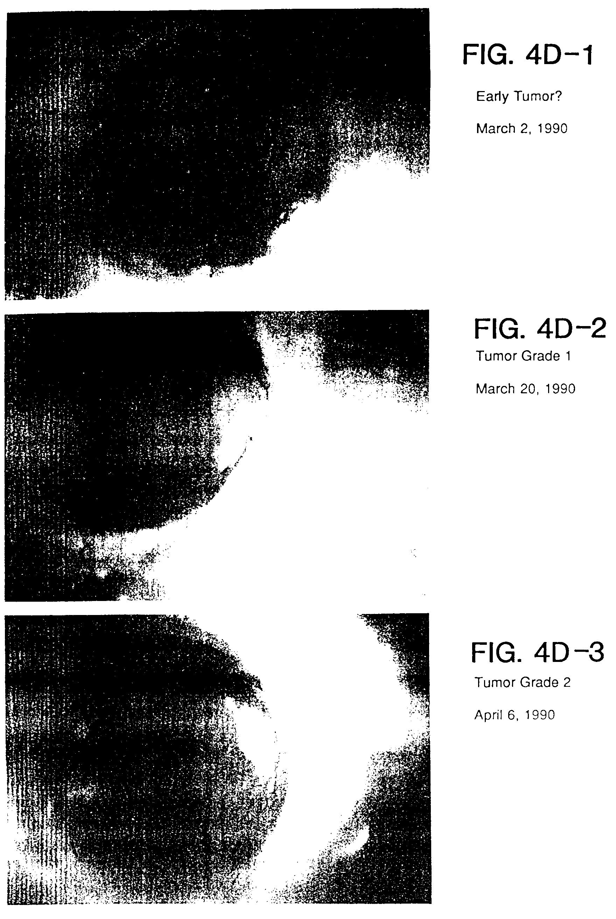

Not all tumor cells can be growth arrested at a single cell level. The osteosarcoma cell line SaOS₂ is a good example. Transfection of the Rb cDNA plasmid HuBAcpr-1-neo-Rb-PQ into this cell line revealed three different cellular responses. Presumably the SaOS₂ cell line consists of more than one cell population. As is shown in Figures 4A and B, 2/3 of the SaOS₂ cells transfected with HuBAcpr-1-neo-Rb-PQ which express the Rb protein failed to divide, while 1/3 did. Approximately 1/3 of the transfected SaOS₂ cells which express Rb protein can divide and form colonies. The remaining 2/3 of the cells that expressed the Rb protein were of two different morphologies. One was small and the other had a cell volume 5-10 times larger. These two populations of cells are present in equal proportions.

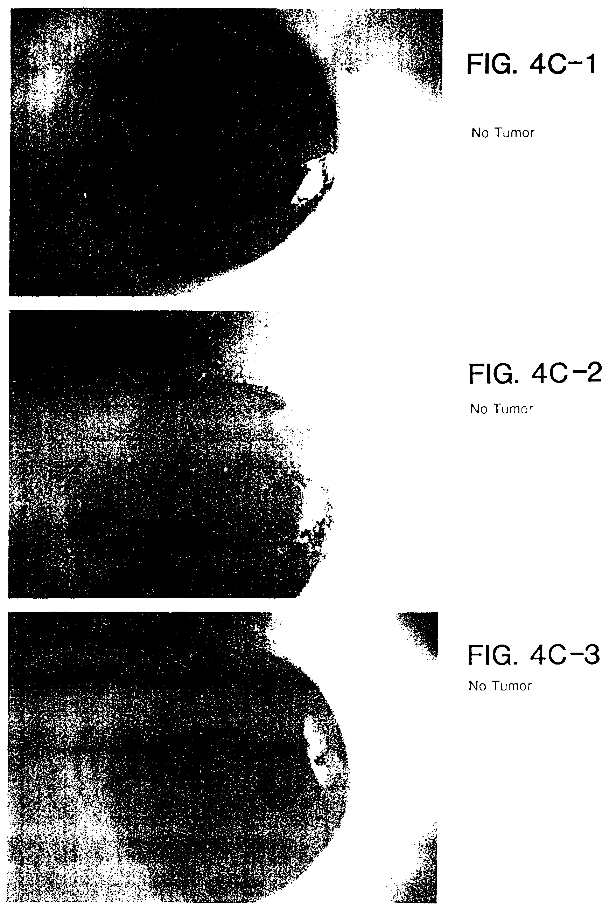

In order to assess whether the SaOS₂ cells that could divide in vitro would grow in vivo, SaOS₂ cells were single-cell cloned by methods known to those of skill in the art. Briefly, for single cell cloning, a colony of the transfected G418 resistant SAOS2 cells was isolated with a cloning cylinder. The cells inside the cloning cylinder were trypsinized and then plated at approximately 200 cells in a 60 mm dish. Cells, attached as single cells on the dish, were grown to approximately 200 cell colonies and the colonies recovered with a cloning cylinder and trypsinized. These cells were then grown up and a sample taken for histoimmunostaining to ensure that all cells stained positive with anti-Rb antibodies Rb1-ABA1 and RB1-AB18. Approximately 10³ cells were then injected into the anterior chamber of the eye of each of ten nude mice. As a control, the parental SaOS₂ cells were injected into the contralateral eye of the same nude mice for comparison. As shown in Figure 4C, SaOS₂ transfected with the Rb construct failed to grow in the eye, whereas the parental SaOS₂ cells proliferated and formed tumors.

### Example 5

### Construction of Growth Inhibiting Ribozyme

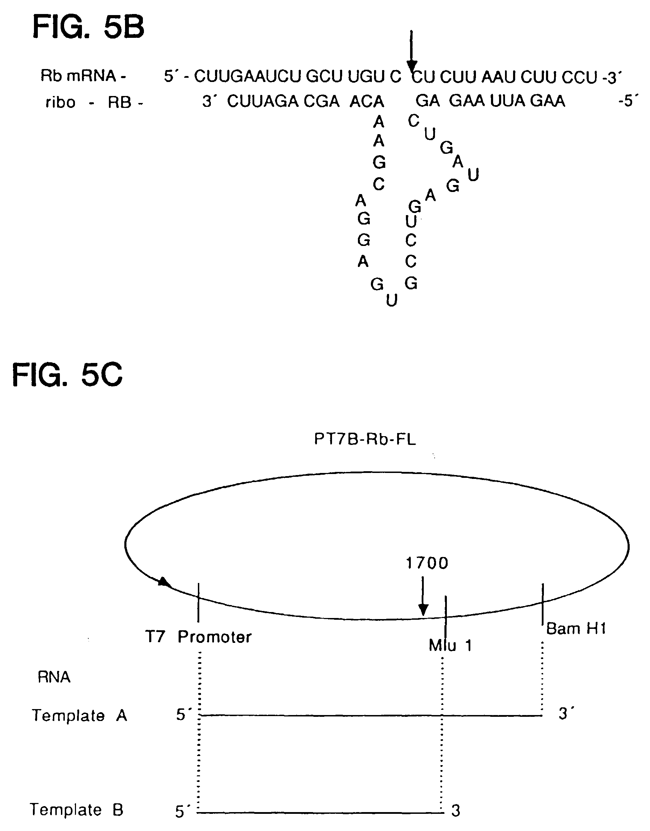

The construction of a DNA construct encoding the ribozyme and further comprising sequences to target the ribozyme to any specific PGR element is schematically presented in generalized form in Figure 5A. The schematic representation of a ribozyme which cleaves the RB-1 gene is also shown in Figure 5B. According to one embodiment of the present invention, a ribozyme construct can be introduced into a cell, which ribozyme will destroy the nuclear or messenger RNA of any PGR element. This aspect is exemplified by the c-myc ribozyme schematically represented in Figure 5D. A portion of the mRNA sequence of the c-myc gene and a designer ribozyme is shown in Figure 5D.

Several ribozymes can be created in a similar fashion for a given mRNA at different positions. The only requirement is the presence of a GUX triplet in the sequence when using the particular ribozyme used in this example. It is clear that other RNA cleaving enzymes may be utilized which have different cleavage sites on the RNA molecule. These may be synthetically prepared or other naturally occurring RNA cleaving molecules.

The ribozyme therefore consists of the catalytic domain 5'-CUG AUG AGU CCG UGA GGA CGA AAC -3' flanked on both the 5' and 3' sides by nucleotide sequences complementary to the 3' and 5' sequence of the mRNA immediately adjacent to the site G U X to be cleaved, X can be either A, C, or U.

In the generalized ribozyme depicted in Figure 5A, N and N' =G, A, U C and N' is complementary to N.B.

### Construction of a Ribozyme Specific for the Rb RNA

The anti-Rb ribozyme which inactivates the Rb mRNA at the cleavage site of the Rb gene between nucleotide 1771 and 1772 was constructed as described below and is shown in Figure 5B.

In order to cut the Rb mRNA, a ribozyme complementary to the Rb mRNA sequence
5'- CUU GAA UCU GCU UGU CCU CUU AAU CUU CCU -3'
was synthesized.

To create the ribo-Rb DNA construct, the following two primers were synthesized:

Sal 1
a) 5' - TCG TCG ACA AGA TTA AGA GCT GAT GAG TCC GTG AGG ACG
3'
b) 5' - CTG GAT CC AAG CCT AAT CTGCT TGT TTC GTC CTC ACG GAC
TC
AT - 3'

One ug each of the two primers was heated to 90°C for 1 minute in 40 ul or 10 mM TRIS HCl pH 7.5, 0.1 mM EDTA. To the heated primers were added 10 ul of 10x primer extension buffer so that the final solution contains 50 mM TRIS HCl pH 7.2, 10 mM MgSO₄, 0.1 mM dithiothreitol, 50 ug/ml bovine serum albumin, 0.1 mM each of dATP, dGTP, dCTP and dTTP. To the mixture was added 4 units of Klenow fragment of DNA polymerase (NE Biolab) and the reaction incubated at room temperature for 1 hour. The reaction was stopped by addition of 1 ul of 0.5 M EDTA and the DNA purified by standard protocols known to those of skill in the art, for instance as described by Maniatis et al. (Maniatis, T., Fuitsch, E.F., and Senbrook, J. (1982) Molecular Cloning: A Laboratory Manual (Cold Spring Harbor Laboratory, Cold Spring Harbor, N.Y., U.S.A.). The purified DNA was cut with Sal1 and BamH1 and subcloned into pGEM4 vector (Promega Biotech) for in vitro expression with T7 polymerase and testing for catalytic activity.

To test the catalytic activity of the ribozyme, RNA was generated by in vitro transcription from the PGEM-ribo-Rb-3 (for ribozyme RNA), and from PT7BRBFL (for substrate RNA) after the plasmids were linearized at the appropriate sites for the desirable length of RNA needed. For in vitro transcription, 2 uM of the linearized plasmid DNA was incubated at 40°C for 1 hour in 50 ul reaction mixture containing 25 units of T7 RNA polymerase, 1 mM ATP, CTP and GTP, 50 units of RNasin, 0.1 mM UTP and 10 uCi of [q-³²P]UTP, 40 mM TRIS HCl pH 7.9, 20 mm MgCl₂, 10 mM NaCl and 10 mM DTT. After the reaction, the ribozyme RNA was purified by electrophoresis on a 6% 7 M urea-polyacrylamide gel. The synthetic ribozyme was identified by exposing the gel to x-ray film for 10 minutes at room temperature.

Gel slices containing the ribozyme were excised and the RNA recovered by vortexing the gel pieces in 0.1% SDS, 0.5 M ammonium acetate pH 6.9, 1 mM EDTA. The eluate was filtered and then precipitated with 0.1 volume of 5M sodium acetate pH 5.3 and 2 volumes of 100% ethanol.

After the reaction was completed, the RNA samples were purified by vortexing in an equal volume of phenol and centrifuged to separate the two phases. The aqueous phase containing the RNA was extracted with ethyl ether. 0.2 volume of 5 M sodium acetate pH 5.3, and 2 volumes of 100% ethanol was then added to the aqueous phase.

Two templates shown in Figure 5C were generated by linearizing the PT7BRBFL at either the BamH1 site (template A) or the Mlu 1 site (template B). Template A is 4.7 kb while template B is 1.8 kb. If the ribozyme cut at nucleotide # 1700 as designed, the size of template A should be significantly reduced, while the size of template B will only be minimally reduced.

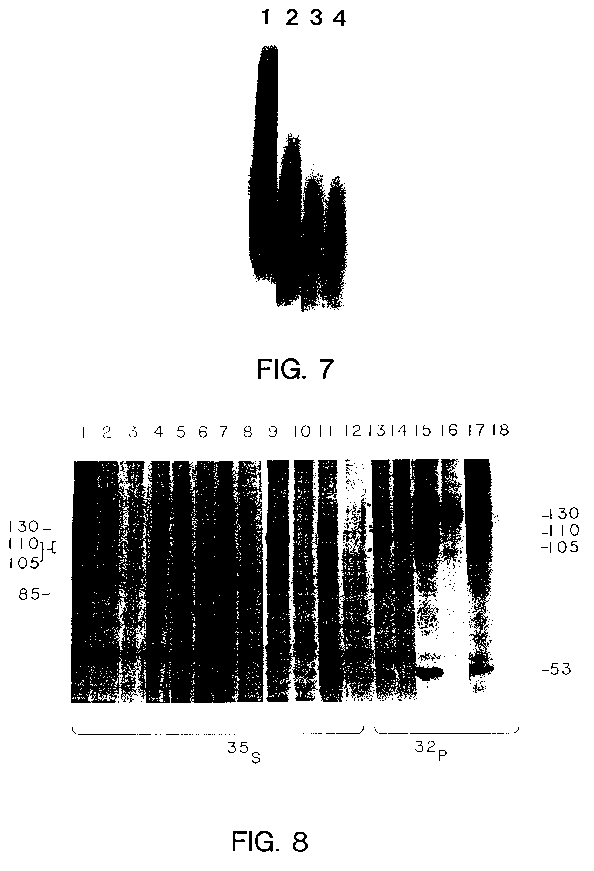

To test the catalytic activity of the ribozyme, 1 pmole of the Rb-ribozyme was mixed either with 1 pmole of template A (Figure 7, lanes 1,2) or of template B (Figure 7, lanes 3,4) in 10 ul of 75 mM TRIS-HCl and 2 mM EDTA. The mixture was heated to 95°C for 2 minutes and then cooled on ice. The reaction was initiated by the addition of MgCl₂ to a final concentration of 20 mM. After 12 hours at 4°C or 42°C, the reaction was stopped by the addition of 2 ul of 200 mM EDTA pH 7.9. The reaction product was analyzed by Formaldehyde agarose gel electrophoresis as previously described (Fung et al (1987) Science 236:1657). As is demonstrated in Figure 7, lane 2, the template A RNA product (lane 1) was degraded to about half its original size as predicted. However, the product size of template B RNA was not appreciably reduced, also as predicted.

Therefore, it is apparent that the Rb-Ribozyme construct cleaved the Rb RNA at the designated site, thereby inactivating the Rb RNA. One can construct according to the above described method a ribozyme directed against any specific RNA, once the sequence of the RNA is deduced. One may also find it useful to construct multiple ribozymes directed against several sites in the same target RNA in order to more efficiently destroy or inactivate the target RNA.

### Construction of a Ribozyme Specific for the c-myc mRNA

In order to cut the c-myc mRNA, a ribozyme complementary to the c-myc sequence
5' -U CCU CAC AGC CCC CUG GUC CUC AAG AGG - 3'
is synthesized. This results in the structure shown in Figure 5D which cuts the target c-myc mRNA at the desired position.

An example of a ribozyme of the c-myc mRNA for the cleavage site between nucleotide 888 and 889 is shown in Figure 5D.

In order to destroy the c-myc RNA in a cell, a DNA plasmid that encodes the ribozyme is needed. The construction of this ribozyme plasmid comprises the following steps:
1. Synthesis of the ribozyme domain.
2. Subcloning of the ribozyme domain into mammalian expression vectors.

### Synthesis of the Ribozyme Domain

The ribozyme for c-myc mRNA shown in Figure 5D is created as follows: Two oligonucleotides are synthesized.

Sal 1
(a) 5' - TC GTC GAC CCT CTT GAG CTG ATG AGT CCG TGA GGA CGA
- 3'
BamH1 HindIII
(b) 5' CT GGA TCC AAG CCT GCC CCC TGG TTT CGT CCT CAC CGA
C- AT - 3'

The underlined nucleotides in (a) are those that are complementary to c-myc mRNA 3' to the cleavage site.

The underlined nucleotides in (b) are those that are the sequences in the c-myc mRNA 5' to the cleavage site.

One ug each of the two primers were heated to 90°C for 1 minute in 40 ul of 10 mM Tris HCl pH. 7.5, 0.1 mM EDTA. To the heated primers was added 10 ul of 10x primer extension buffer so that the final solution contains 50 mM Tris HCl pH 7.2, 10 mM MgSO₄, 0.1 mM dithiothreitol, 50 ug/ml bovine serum albumin, 0.1 mM each of dATP, dGTP, dCTP and dTTP. To the mixture was added 4 units of Klenow fragment of DNA polymerase (NE Biolab) and the reaction incubated at room temperature for 1 hour. The reaction was stopped by addition of 1 ul of 0.5 M EDTA and the DNA purified by standard protocols. The purified DNA was cut with Sal 1 and BamH1 and was subcloned into PGEMA vector (Promega diatech) for in vitro expression with T7 polymerase and testing of catalytic activity (see below). Alternatively, the purified DNA may be subcloned into Sal 1/BamH1 sites of HBApr-1-neo to give:
HuBAcpr-1-neo-Ribo-c-myc-1

### Example 6

### Generation of Retrovirus from the Recombinant Construct

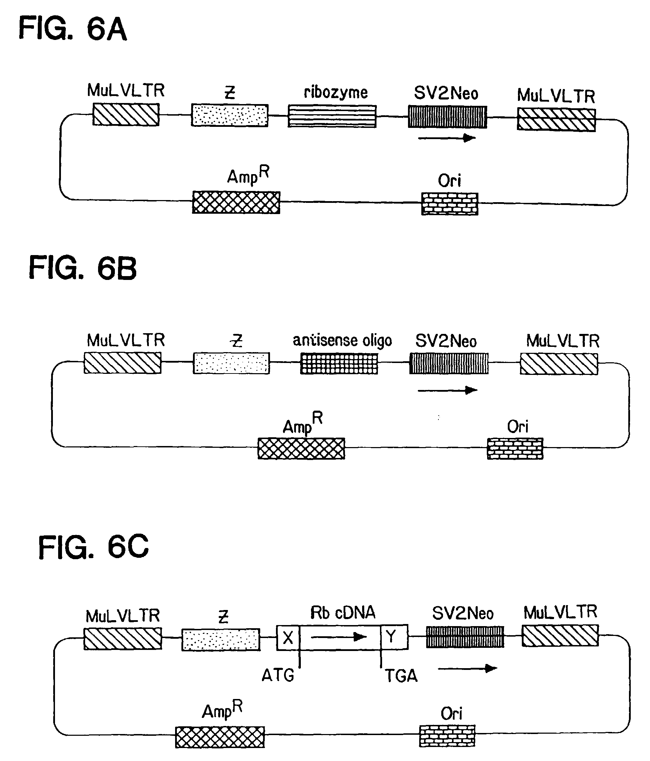

In order to deliver the ribozyme construct HuBAcpr-1-neo-ribo-c-myc-1 into cells with a retroviral vector, the EcoR1/Hind III fragment was excised and subcloned into the EcoR1/Hind III sites of the Murine Leukemia virus based retroviral vector PMV7. The structure of the resulting retroviral construct PMV7-HBAp-ribo-c-myc-1 is shown in Figure 6A.

Figure 6A shows a schematic representation of a ribozyme in an MuLV vector for the c-myc mRNA, where Z can be (1) a short stretch of synthetic linkers for subcloning of the ribozyme into the MuLV vector, or (2) the human β-actin promoter. The ribozyme structure is as in Figure 5D.

Retroviral plasmid DNA obtained for the ribozyme or antisense or one with the negatively acting growth regulatory genes is transfected into ψm or PA12 cells by electroporation and G418 resistant colonies isolated as described in Example 2. Tissue culture supernatant is harvested daily and clarified by centrifugation at 4°C 10,000 rpm in a sorval HB-4 rotor. The virus titer is determined by incubating NIH 3T3 cells with serial dilutions of the supernatant. The cells are grown in RPMI 1640 medium plus 10% FCS and 100 ug/ml G418. The virus titer is determined from the number of G418 resistant colonies obtained. If only a low titer is obtained, the virus will be used to infect ψ2 cells and the supernatant from the G418 resistant ψ2 cells analyzed for determination of viral titer as above. If the virus titer is still low, the supernatant from ψ2 will be used to infect uninfected ψm cells and the supernatant from cm will be tested for viral titer. Using this protocol of infection, a viral titer of 10³-10⁵/ml is usually obtained. The cell colonies expressing a high titer 10³⁻⁵/ml will be grown up for the production of virus. The virus will then be used for therapeutic purpose.

An alternative vector for the ribozyme has been described (Cotten M. and Birnstiel M.L. (1989) EMBO J 8:3861). The ribozyme was subcloned into the structural gene of tRNA^{Met}. The resulting hybrid molecule ribo-tRNA^{Met} was found to have catalytic activity for its intended RNA substrate. This and other ribozyme systems may also be used. (Kuo, et al. (1988) J. Virol 62:4439; Herschlag and Cech (1990) Nature 344 7:405; Robertson and Joyce (1990) Nature 344:467).

### Example 7

### Analysis of Ribozyme Catalytic Activity In Vitro and In Vivo

To generate ribozyme in vitro, 2 uM of the ribozyme plasmid pGEM-ribo-c-myc-1 was incubated at 40°C for 1 hour in 50 ul reaction solution containing 0.5 unit/ul T7 RNA polymerase; 40 mM TRIS-HCl pH 7.9, 20 mM MgCl₂; 10 mM NaCl; 10 mM DTT, 0.1 mM UTP, 10 uCi of [γ³²P]UTP, 1 mM each of ATP, CTP and GTP; and 1 unit/ul of human placental RNase inhibitor.

After the reaction was completed, 50 ul of loadings solution containing 95% formamide, 1 mM EDTA, 0.1% (w/v) xylene cyanol blue, 0.1% (w/v) bromophenol blue was added to the reaction mixture.

The solution was heated at 65°C for 5 minutes and then loaded onto a 6% 7M urea-polyacrylamide gel. Electrophoresis was done in 1x TBE buffer (50 mM Tris-borate pH 8.4 and 1.5 mM EDTA). The synthetic ribozyme was located by exposure of an X-ray film to the gel at room temperature for 10 minutes. Gel slices containing the ribozyme were excised and the RNA eluted by vortexing in 0.1% SDS, 0.5 M ammonium acetate pH 6.9, 1 mM EDTA. The eluate was filtered and then precipitated with 0.1 volume of 5M sodium acetate pH 5.3 and 2 volumes of 100% ethanol.

To test the catalytic activity of the ribozyme, 1 pmol of the ribozyme isolated above was mixed with 1 pmol of c-myc mRNA in 75 mM TRIS-HCl and 2 mM EDTA. The mixture was heated to 95°C for 2 minutes and then quickly cooled on ice. The reaction was initiated by adjusting to a final volume of 10 ul containing 50 mM TRIS-HCl pH 7.5, 1 mM EDTA and 10-20 mM MgCl₂ for 12 hours at 37°C under a layer of paraffin oil. The reaction was stopped by the addition of 2 ul of 200 mM EDTA pH 7.9. The reaction product was analyzed by Northern Blot.

### Example 8

### Immunohistochemical Localization of the Rb Protein in Cells

Cells were cultured in RPM1 1640 medium supplemented with 10% FCS and 0.1% Penicillin/streptomycin. The cells were rinsed twice with cold PBS (phosphate buffered saline). The cells were fixed with a solution containing 5% acetic acid and 95% ethanol at 4°C for 12 hours.

After fixing, the cells were washed twice in cold PBS. The nonspecific binding was blocked by incubating the cells in a PBS solution containing 1% normal horse serum, 3% bovine serum albumin, and 0.2% Triton X-100 for 1 hour at 4°C. The cells were then rinsed twice with PBS. The cells were then incubated with either a 4°C solution containing a 1:1600 dilution of RB1-AB18 or a 1:100 dilution of RB1-ABA1 in PBS for 1 hour at 37°C. The cells were then washed sequentially with cold PBS and PBS containing 1% Triton X-100 and PBS. The specific binding of the RB1 antibodies to the cells was visualized by incubation with biotinylated goat anti-rabbit IgG antibodies (diluted 1:200 in PBS) for 1 hour at 37°C. After washing the cells three times with cold PBS, the cells were incubated with ABC reagent (avidin: biotin complex) at 37°C for 30 minutes, washed with cold PBS and then incubated with freshly prepared DAB (3,3'-Diamixobenzidine - tetrahydrochloride) solution (6mg of DAB in 10 ml of 0.05 M TRIS-HCl, pH 7.6, 0.3% sodium azide and 10ul hydrogen peroxide) at room temperature for 4 minutes. The cells were then washed with distilled water and examined under a light microscope.

### Example 9

### Antisense RNA Constructs for C-myc and C-myb

The uses of antisense RNA for c-myc and c-myb to inhibit the growth of the non-lymphocytic leukemia cell line HL-60 and other cell lines have been described previously (Wackstrom et al (1988) Proc. Natl. Acad. Sci 85:1028; Anfossi et al. (1989) Proc. Natl. Acad. Sci 86:3379). These experiments were done by incubating cells in vitro with the oligoribonucleotide. For in vivo use, a pair of oligonucleotides for a given antisense RNA is produced as follows: For c-myc, a sequence complimentary to the first 15 bases of the open reading frame (nucleotide # 559-573 of the c-myc mRNA) is flanked by an EcoR1 site on the 5' end and a Hind III site on the 3' end

5' AATTCAACGTTGAGGGCATA 3'
3' ____GTTGCAACTCCCCGTATTCGA 5'
EcoR1 Hind III

The pair of oligonucleotides is heated at 90°C for 1 minute and then annealed in 2x ligation buffer (20 mM TRIS HCl pH 7.5, 10 mM MgCl₂, 10 MM dithiothreitol (DTT) and 0.2 mM ATP) and then ligated to the EcoR1/Hind III site of the retroviral vector PMV7.

The cDNA coding for antisense RNA for positively acting growth regulatory genes (for example c-fos, PDGF receptor etc) is constructed in a similar fashion.

### Example 10

### Immunological Detection of Novel Proteins with Similar or Identical Structural Domains to the Rb Protein

The inventors have recently discovered a group of proteins which share similar or identical structural domains with the Rb protein. These proteins could be products of new negatively acting growth regulatory genes. These proteins were detected by immunoprecipitation with antibodies raised against the RB gene product. Immunoprecipitation was done with anti-Rb antibodies RB1AB 20 and RB1AB 18 and RB1AB A1. The preparation and use of these three antibodies have previously been documented (Mihara et al (1989) Science 246: 1300). In addition, rabbit polyclonal antibodies RB1AB C have been prepared against the peptide P3 (CRTPRRGQNRSARIAKQLEND, a peptide consisting of Rb protein, amino acid number 250 to 270 with a cysteine residue added to the N-terminus).

For immunoprecipitation, a random population of confluent human carcinoma cells SW613 (4 x 10⁶ cells) was incubated in Dulbecco's minimum essential medium (DMEM) and 5% dialyzed fetal bovine serum (FBS) either in the absence of methionine (if [³⁵S] methionine was used as the label) or phosphate (if ³²P phosphate was used). After incubation at 37°C for 1 hour, the medium was replaced with 2 ml of DMEM containing 5% dialyzed FBS and either 300 UCi of [³⁵S] methionine (1000 Ci/mmol) or 300 uCi of [³²P] orthophosphate (9120 Ci/mmol) for 2 hours at 37°C. The cells were then washed twice with phosphate-buffered saline (PBS) and proteins were extracted with 0.5 ml of EBC (40 mM TRIS-HCl pH 8.0, 120 mM NaCl, 0.5% NP-40, aprotinine (2 ug/ml), pepstatin (2 ug/ml), leupeptin (2 ug/ml) and phenylmethylsulfonyl fluoride (100 ug/ml) at 0°C for 1 hour. The cell lysates were clarified by centrifugation at 15,000 rpm for 10 minutes at 4°C. The supernatant was incubated for 2 hours at 4°C with either 5-10 ul of the anti-Rb antibodies or antibodies that had been incubated with 100 ug of the immunizing peptide for 2 hours or more at 4°C. The immunocomplex was collected on protein A-agarose (Calbiochem) and washed 5 times with EBC. The immunoprecipitated proteins were eluted by heating for 10 minutes at 87°C in sample buffer [62.5 mM TRIS HCl pH 6.8, 5% β-mercaptoethanol, 2.3% SDS, 10% glycerol, and 0.001% Bromophenol blue] and separated by SDS-PAGE. The gel was fixed and exposed to X-ray film.

As can be seen in Figure 8, there are at least four groups of proteins that can be precipitated with anti Rb antibodies. These proteins must share a similar structural domain with the Rb protein and are therefore recognized by the antibodies against the Rb protein. Other proteins may be associated with the Rb protein and be co-precipitated. The characteristics of these proteins are listed below.
(1) P110 - P130 is a heterogenous group of protein species that has molecular weight ranging from 110 to 130 kd. The molecular weights were best estimated from the migration of gel electrophoresis markers used. The proteins could have molecular weights ranging from 107 to 150 kd.
   These proteins were first detected in the human carcinoma cell line SW613 which has Rb protein. As can be seen in Figure 8, lanes 1, 9 and 13, RB1AB 20 precipitated a group of proteins in the molecular weight range 105-130 kilodalton (kd). That these protein are specifically recognized by the anti Rb antibodies is shown by the fact that preabsorbing the antibodies with the immunizing peptide P5 leads to failure to immunoprecipitate these proteins (Figure 8, lanes 2, 10 and 14). Instead of being associated with the Rb protein as a complex, this group of proteins appears to share a structural motif with the Rb protein. This is shown by the fact that they are detected with RB1AB 20 in cells such as the breast tumor cell line BT-549 and the retinoblastoma cell line Y79 (Figure 8, lane 11), both of which have no endogenous Rb protein. In addition, this shows that these proteins are not encoded by the Rb 1 gene.
   It is possible that one or more members of this group of proteins may be related to the p107/p120 protein recently reported (Ewen M.E. et al (1989) Cell 58:257; Dyson N. et al 1989) Cell 58:249). P107/p120 is a protein species that can bind to the large T antigens of SV40 and JC virus as well as to the E1A protein of adenovirus. Since the same viral antigen domains were used to bind these and the Rb protein, these proteins may share structural motif that can be recognized by these viral proteins. Deletion mapping of the Rb gene has located the stretches of amino acids #393-572 and #646-772 important for binding to these viral antigens. In fact antibodies raised against these regions of the Rb protein can recognize the p107/p120 species. However, it should be noted that P5, the stretch of amino acid to which RB1AB 20 was raised against is outside the domain required for binding to E1A or SV40 Large T. P5 may be part of the domain that is also conserved among these proteins. P107-110 kd, also recognized by the antibodies, is shown in Figure 8, lanes 3 and 4. Immunoprecipitation of cell lysate from the cervical carcinoma cell line ME18O showed the presence of the 105 Kd and 110 Kd protein in Figure 8, lane 3. Preabsorbing the antibodies with peptide P5 resulted in disappearance of both the bands. However, because heavily phosphorylated Rb 105 Kd protein has a molecular weight of about 107-110 Kd, it may obscure other proteins in that region. In order to test whether these proteins were obscured by the phosphorylated forms of the Rb protein, the same experiment was carried out using the cervical carcinoma cell line C33A (Figure 8, lanes 5 and 6) and the bladder cell line J82 (Figure 8, lanes 7 and 8), in which the Rb protein is mutated and cannot be phosphorylated. Immunoprecipitation of cell lysates from both cell lines showed the presence of the 105 Kd unphosphorylated form of Rb protein as well as a 107 Kd protein. Therefore, the RB1AB-20 can bring down a group of proteins in the molecular weight range of 107-130 kd. These proteins share a common structural domain with the Rb protein and are recognizable by the anti Rb antibodies.
(2). A protein P85 having a molecular weight of approximately 85kd, has been detected in the human carcinoma cell line SW613. P85 can be detected using the antibodies RB1AB 20 and RB1AB C. As shown in Figure 8, lanes 1 and 2, P85 can be readily detected (Figure 8, lane 1), but not if the antibodies were blocked with the immunizing peptide (P5) (Figure 8, lane 2). When proliferating normal human keratinocytes were treated with TGF-β, growth arrest is associated with the upregulation of expression of the P85 protein. More importantly, enhancement of the expression of P85 is dependent on the dosage and duration of treatment with TGF-β. It is well known that TGF-β treatment can result in growth inhibition of many cell types. The fact that P85 expression in cells is enhanced by TGF-β treatment is consistent with the possibility that P85 is another negatively acting growth regulatory gene product that shares a common structural motif with the Rb protein.
(3). Another protein P53 with a molecular weight of approximately 53 kd was precipitated by the RB antibodies. Perhaps the strongest proof that the proteins that share a common structural motif with the Rb protein are all negatively-acting growth regulatory gene products is the finding that the tumor suppressor gene product P53 is precipitated by the RB1AB 20 (Figure 8, lanes 13 and 14) and RB1AB18 (Figure 8, lanes 15-18) antibodies. In order to demonstrate that P53 is not coprecipitated due to an association or linkage with Rb, the human breast carcinoma cell line MDAMB468, in which the Rb gene was deleted, but which contains the P53 gene was tested. P53 was also precipitated from the human breast carcinoma cell line MDAMB468 with both of the antibodies.

Thus, these groups of proteins may be encoded by negatively acting growth regulatory genes. These genes can therefore also be used in practicing the present invention for the suppression of cell growth by introducing them into abnormally proliferating target cells.

The invention now being fully described, it will be apparent to one of ordinary skill in the art that many changes and modifications can be made thereto without departing from the spirit or scope of the invention as set forth below.

## Claims

1. A pharmaceutical composition for the treatment of pathological cellular proliferative diseases by administration of said composition to a cell, said composition comprising a negative growth regulatory gene which directs the expression of an encoded peptide.

2. A pharmaceutical composition according to claim 1, wherein said DNA encoding a negative growth regulatory gene is contained within a recombinant expression vector.

3. A pharmaceutical composition according to claim 2, wherein said expression vector is a viral vector.

4. A pharmaceutical composition according to claim 2, wherein said expression vector is an adenoviral vector.

5. A pharmaceutical composition according to claim 2, wherein said expression vector is a plasmid.

6. A pharmaceutical composition according to claim 5, wherein said plasmid is encapsulated in a liposome.

7. A pharmaceutical composition according to claim 2, wherein said negative growth regulatory gene is selected from the group consisting of the p53 gene, the p85 gene, the p107 gene, the p130 gene, the DCC gene and the NF-1 gene.

8. A pharmaceutical composition according to claim 7, wherein said negative growth regulatory gene is the p53 gene.

9. A pharmaceutical composition according to claim 1, wherein the treatment is of abnormally proliferating cells associated with a disease selected from the group consisting of psoriasis, benign proliferative skin diseases, ichthyosis, papilloma, basal cell carcinoma, squamous cell carcinoma, fibroproliferative diseases such as restenosis, vasoproliferative diseases, dermatoproliferative diseases, and neoplastic diseases of the eye.

10. A pharmaceutical composition according to claim 1, wherein said proliferating cells contain at least one active allele of a negative growth regulatory gene but still proliferate inappropriately.

11. A pharmaceutical composition according to claim 1, wherein the treatment is for inhibiting the growth of proliferative cells in which the endogenous negative growth regulatory genes are inactive or absent or the expression of the negative regulatory genes or their gene products is inhibited.

12. A pharmaceutical composition according to claim 11, wherein said proliferating cells have mutated endogenous negative regulatory gene.

13. Use of DNA encoding a negative growth regulatory gene which directs the expression of an encoded peptide, in the preparation of a medicament for use in a method of treatment of pathological cellular proliferative diseases by administration of said medicament to a cell.

14. Use according to claim 13, wherein said DNA encoding a negative growth regulatory gene is contained within a recombinant expression vector.

15. Use according to claim 14, wherein said expression vector is a viral vector.

16. Use according to claim 14, wherein said expression vector is an adenoviral vector.

17. Use according to claim 14, wherein said expression vector is a plasmid.

18. Use according to claim 17, wherein said plasmid is encapsulated in a liposome.

19. Use according to claim 14, wherein said negative growth regulatory gene is selected from the group consisting of the p53 gene, the p85 gene, the p107 gene, the p130 gene, the DCC gene, and the NF-1 gene.

20. Use according to claim 19, wherein said negative growth regulatory gene is the p53 gene.

21. Use according to claim 13, wherein the treatment is of abnormally proliferating cells associated with a disease selected from the group consisting of psoriasis, benign proliferative skin diseases, ichthyosis, papilloma, basal cell carcinoma, squamous cell carcinoma, fibroproliferative diseases such as restenosis, vasoproliferative diseases, dermatoproliferative diseases, and neoplastic diseases of the eye.

22. Use according to claim 13, wherein said proliferating cells contain at least one active allele of a negative growth gene but still proliferate inappropriately.

23. Use according to claim 13, wherein the treatment is for inhibiting the growth of proliferating cells in which the endogenous negative growth regulatory genes are inactive or absent or the expression of the negative regulatory genes or their gene products is inhibited.

24. Use according to claim 23, wherein said proliferating cells have a mutated endogenous negative growth regulatory gene.
